# EUROPEAN PATENT SPECIFICATION

(11) **EP 1 635 828 B1**
(45) Date of publication and mention of the grant of the patent: **23.04.2008**
(21) Application number: 04725298.6
(22) Date of filing: 02.04.2004
(51) Int. Cl.: A61K 31/451, A61K 31/4418, C07D 211/22, C07D 211/70, C07D 407/12, A61P 25/00

(54) **4-(2-PHENYLOXYPHENYL)-PIPERIDINE OR -1,2,3,6-TETRAHYDROPYRIDINE DERIVATIVES AS SEROTONIN REUPTAKE INHIBITORS**
4-(2-PHENYLOXYPHENYL)-PIPERIDIN- ODER -1,2,3,6-TETRAHYDROPYRIDIN-DERIVATE ALS SEROTONIN-WIEDERAUFNAHME-HEMMER
DERIVES DE 4-(2-PHENYLOXYPHENYL)-PIPERIDINE OU 1,2,3,6-TETRAHYDROPYRIDINE UTILISES COMME INHIBITEURS DE LA RECAPTURE DE LA SEROTONINE

(30) Priority: 04.04.2003 DK 200300519; 04.04.2003 US 460265 P
(43) Date of publication of application: 22.03.2006
(73) Proprietor: H. LUNDBECK A/S, 2500 Valby-Copenhagen (DK)
(72) Inventor: BANG-ANDERSEN, Benny, Copenhagen S, 2300 (DK); KROLL, Friedrich, 2800 Mechelen (BE); KEHLER, Jan, Kgs. Lyngby, 2800 (DK)
(86) International application number: PCT/DK2004/000241
(87) International publication number: WO 2004/087155

(56) References cited:
- WO-A-01/27068
- WO-A-03/029232
- US-A- 4 198 417
- US-A- 4 241 071
- L. MARTIN ET AL.: "Synthesis of Spiro[isobenzofuran-1(3H),4'piperidines] as Potential Central Nervous System Agents. Conformationally Mobile Analogues Derived by Furan Ring Opening" J. MED. CHEM., vol. 22, no. 11, 1979, pages 1347-1354, XP002286159

## Description

The present invention relates to novel compounds which are serotonin reuptake inhibitors and as such effective in the treatment of for example depression and anxiety.

### Background of the invention

Antidepressants are disclosed in e.g. US 4 198 417 and US 4 241 071.

Selective serotonin reuptake inhibitors (hereinafter referred to as SSRIs) have become first choice therapeutics in the treatment of depression, certain forms of anxiety and social phobias, because they are effective, well tolerated and have a favourable safety profile compared to the classic tricyclic antidepressants.

However, clinical studies on depression indicate that non-response to SSRIs is substantial, up to 30%. Another, often neglected, factor in antidepressant treatment is compliance, which has a rather profound effect on the patient's motivation to continue pharmacotherapy.

First of all, there is the delay in therapeutic effect of SSRIs. Sometimes symptoms even worsen during the first weeks of treatment. Secondly, sexual dysfunction is a side effect common to all SSRIs. Without addressing these problems, real progress in the pharmacotherapy of depression and anxiety disorders is not likely to happen.

The combined effect of serotonin reuptake inhibition and norepinephrine uptake inhibition on depression is explored in clinical studies of compounds such as Duloxetine (Wong, Duloxetine (LY-248686): an inhibitor of serotonin and noradrenaline uptake and an antidepressant drug candidate Expert Opinion on Investigational Drugs, 1998, 7, 10, 1691-1699) and Venlafaxine (Khan-A et al, Venlafaxine in depressed outpatients Psychopharmacology Bulletin, 1991, 27, 141-144).

The present invention provides novel compounds which posses the combined effect of serotonin reuptake inhibition and norepinephrine uptake inhibition for the treatment of affective disorders, such as depression, anxiety disorders including general anxiety disorder, social anxiety disorder, post traumatic stress disorder, obsessive compulsive disorder, panic disorder, panic attacks, specific phobias, social phobia and agoraphobia.

### Summary of the invention

The present invention provides compounds of the general formula I 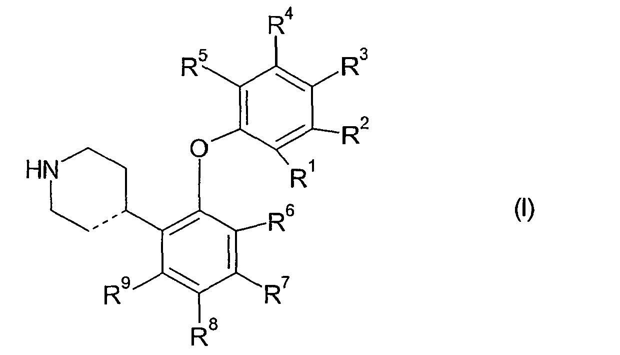 wherein the dotted line, R¹, R², R³, R⁴, R⁵, R⁶, R⁷, R⁸, and R⁹ are as defined below.

The invention provides a compound according to the above for use as a medicament.

The invention provides a pharmaceutical composition comprising a compound according to the above or a pharmaceutically acceptable acid addition salt thereof and at least one pharmaceutically acceptable carrier or diluent.

The invention provides the use of a compound according to the above or a pharmaceutically acceptable acid addition salt thereof for the preparation of a medicament for the treatment of affective disorders, such as depression, anxiety disorders including general anxiety disorder, social anxiety disorder, post traumatic stress disorder, obsessive compulsive disorder, panic disorder, panic attacks, specific phobias, social phobia and agoraphobia.

The invention provides a method for the treatment of an affective disorders, such as depression, anxiety disorders including general anxiety disorder, social anxiety disorder, post traumatic stress disorder, obsessive compulsive disorder, panic disorder, panic attacks, specific phobias, social phobia and agoraphobia in a living animal body, including a human, comprising administering a therapeutically effective amount of a compound according to the above or a pharmaceutically acceptable acid addition salt thereof.

### Definition of substituents

Halogen means fluoro, chloro, bromo or iodo.

The expression C₁₋₆-alk(en/yn)yl means a C₁₋₆-alkyl, C₂₋₆-alkenyl or a C₂₋₆-alkynyl group.

The term C₁₋₆ alkyl refers to a branched or unbranched alkyl group having from one to six carbon atoms inclusive, including but not limited to methyl, ethyl, 1-propyl, 2-propyl, 1-butyl, 2-butyl, 2-methyl-2-propyl and 2-methyl-1-propyl.

Similarly, C₂₋₆ alkenyl and C₂₋₆ alkynyl, respectively, designate such groups having from two to six carbon atoms, including one double bond and one triple bond respectively, including but not limited to ethenyl, propenyl, butenyl, ethynyl, propynyl and butynyl.

The terms C₁₋₆-alk(en/yn)yloxy, C₁₋₆ alk(en/yn)ylsulfanyl, hydroxy-C₁₋₆-alk(en/yn)yl, halo-C₁₋₆-alk(en/yn)yl, cyano-C₁₋₆-alk(en/yn)yl, NR^{z}R^{w}-C₁₋₆-alk(en/yn)yl, C₁₋₆-alk(en/yn)yloxy-C₁₋₆-alk(en/yn)yl and halo-C₁₋₆-alk(en/yn)yloxy designate such groups in which the C₁₋₆-alk(en/yn)yl are as defined above. Halo means halogen. NR^{z}R^{w}-C₁₋₆-alk(en/yn)yl designate the group

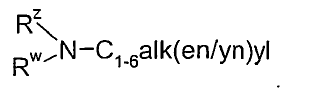

The term C₃₋₈ cycloalkyl designates a monocyclic or bicyclic carbocycle having three to eight C-atoms, including but not limited to cyclopropyl, cyclopentyl, cyclohexyl, etc.

The term C₃₋₈ cylcoalkenyl designates a monocyclic or bicyclic carbocycle having three to eight C-atoms and including one double bond.

In the term C₃₋₈-cycloalk(en)yl-C₁₋₆-alk(en/yn)yl, C₃₋₈-cycloalk(en)yl and C₁₋₆-alk(en/yn)yl are as defined above.

The term 3-7-membered ring optionally containing one further heteroatom, such as N, O, or S, as used herein refers to ring systems such as 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, all of which may be further substituted with a group selected from a C₁₋₆-alk(en/yn)yl, hydroxy, hydroxy-C₁₋₆-alk(en/yn)yl, C₁₋₆-alk(en/yn)yloxy-C₁₋₆-alk(en/yn)yl.

### Description of the invention

The present invention relates to 4-(2-phenyloxyphenyl)-piperidine or -1,2,3,6-tetrahydropyridine derivatives which are serotonin reuptake inhibitors and as such effective in the treatment of for example depression and anxiety. In particular the piperidines are also good norepinephrine uptake inhibitors.

Accordingly the present invention relates to a compound represented by the general formula I 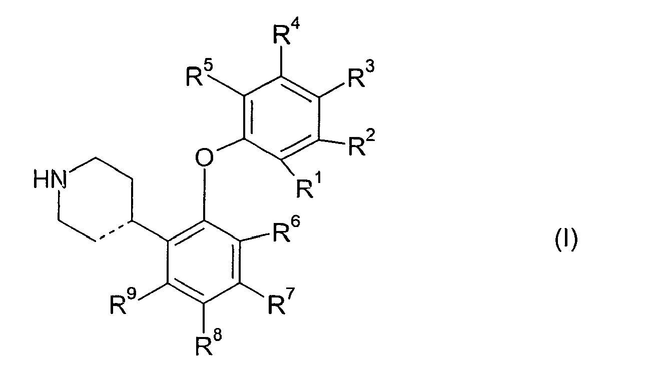 wherein
the dotted line ---- indicates a single bond or a double bond;
R¹, R², R³, R⁴, R⁵ are independently selected from hydrogen, halogen, cyano, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; or
R² and R³ together form a heterocycle fused to the phenyl ring selected from 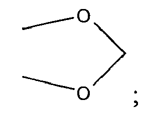 and R¹, R⁴, R⁵ are as defined above;
R⁶, R⁷, R⁸, R⁹are independently selected from hydrogen, halogen, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom;
provided that at least one of R¹, R², R³, R⁴, R⁵, R⁶, R⁷, R⁸, and R⁹ is different from hydrogen;
or a salt thereof.

In one embodiment of the compound of formula I, R¹ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom. In a further embodiment of the compound of formula I R¹ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. In a further embodiment R¹ is NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk-(en)yl-C₁₋₆-alk(en/yn)yl, such as hydrogen, cyanomethyl, C₁₋₆-alk(en/yn)yl. In a further embodiment R¹ is NR^{x}R^{y} wherein R^{x} is NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, and R^{y} is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl. In a further embodiment R¹ is NR^{x}R^{y} wherein R^{x} and R^{y} together with the nitrogen to which they are attached form a 3-7-membered ring which optionally contains one further heteroatom, such as 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or more selected from a C₁₋₆-alk(en/yn)yl, hydroxy, hydroxy-C₁₋₆-alk(en/yn)yl, C₁₋₆-alk(en/yn)yloxy-C₁₋₆-alk(en/yn)yl, e.g. one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. Typically, R¹ is selected from hydrogen; halogen; cyano; C₁₋₆-alkyl; C₁₋₆-alkyloxy; C₁₋₆-alkylsulfanyl; halo-C₁₋₆-alkyl; NR^{x}R^{y} wherein R^{x} and R^{y} are independently selected from hydrogen, C₁₋₆-alkyl, cyanomethyl; NR^{x}R^{y} wherein R^{y} is selected from hydrogen, or C₁₋₆-alkyl, and R^{x} is NR^{z}R^{w}-C₁₋₆-alk(en/yn)yl wherein R^{z} and R^{w} are independently selected from hydrogen, or C₁₋₆-alkyl; 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. To further illustrate without limiting the invention an embodiment of R¹ is hydrogen; another embodiment of R¹ is C₁₋₆-alkyl, such as methyl; a further embodiment of R¹ is halogen, such as fluoro, or chloro.

In a further embodiment of the compound of formula I, R² is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. Typically, R² is selected from hydrogen, halogen, cyano, C₁₋₆-alkyl, C₁₋₆-alkyloxy, C₁₋₆-alkylsulfanyl, halo-C₁₋₆-alkyl. To further illustrate without limiting the invention an embodiment of R² is hydrogen; another embodiment of R² is C₁₋₆-alkoxy, such as methoxy; another embodiment of R² is halo-C₁₋₆-alkyl, such as trifluoromethyl; another embodiment of R² is C₁₋₆-alkyl, such as methyl; another embodiment of R² is halogen, such as chloro.

In a further embodiment of the compound of formula I, R³ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. Typically, R³ is selected from hydrogen, halogen, cyano, C₁₋₆-alkyl, C₁₋₆-alkyloxy, C₁₋₆-alkylsulfanyl, halo-C₁₋₆-alkyl. To further illustrate without limiting the invention an embodiment of R³ is hydrogen; another embodiment of R³ is C₁₋₆-alkyl, such as methyl; a further embodiment of R³ is C₁₋₆-alkoxy, such as methoxy; a further embodiment of R³ is halogen, such as chloro, or fluoro; another embodiment of R³ is halo-C₁₋₆-alkyl, such as trifluoromethyl.

In a further embodiment of the compound of formula I, R² and R³ together form a heterocycle fused to the phenyl ring selected from

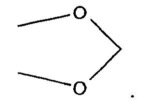

In a further embodiment of the compound of formula I, R⁴ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. Typically, R⁴ is selected from hydrogen, halogen, cyano, C₁₋₆-alkyl, C₁₋₆-allcyloxy, C₁₋₆-alkylsulfanyl, halo-C₁₋₆-alkyl. To further illustrate without limiting the invention an embodiment of R⁴ is hydrogen; another embodiment of R⁴ is C₁₋₆-alkoxy, such as methoxy; another embodiment of R⁴ is halo-C₁₋₆-alkyl, such as trifluoromethyl; another embodiment of R⁴ is C₁₋₆-alkyl, such as methyl; another embodiment of R⁴ is halogen, such as chloro.

In a further embodiment of the compound of formula I, R⁵ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloak(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cyaoalk(en)yl-C₁₋₆-alk(en/yn)yl; or R^{x} and R^{y} together with the nitrogen to which they are attached form a 3-7-membered ring which optionally contains one further heteroatom. In a further embodiment of the compound of formula I R⁵ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl. In a further embodiment R⁵ is NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, such as hydrogen, cyanomethyl, C₁₋₆-alk(en/yn)yl. In a further embodiment R⁵ is NR^{x}R^{y} wherein R^{x} is NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, and R^{y} is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl. In a further embodiment R⁵ is NR^{x}R^{y} wherein R^{x} and R^{y} together with the nitrogen to which they are attached form a 3-7-membered ring which optionally contains one further heteroatom, such as 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or more selected from a C₁₋₆-alk(en/yn)yl, hydroxy, hydroxy-C₁₋₆-alk(en/yn)yl, C₁₋₆-alk(en/yn)yloxy-C₁₋₆-alk(en/yn)yl, e.g. one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. Typically, R⁵ is selected from hydrogen; halogen; cyano; C₁₋₆-alkyl; C₁₋₆-alkyloxy; C₁₋₆-alkylsulfanyl; halo-C₁₋₆-alkyl; NR^{x}R^{y} wherein R^{x} and R^{y} are independently selected from hydrogen, C₁₋₆-alkyl, cyanomethyl; NR^{x}R^{y} wherein R^{y} is selected from hydrogen, or C₁₋₆-alkyl, and R^{x} is NR^{z}R^{w}-C₁₋₆-alk(en/yn)yl wherein R^{z} and R^{w} are independently selected from hydrogen, or C₁₋₆-alkyl; 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. To further illustrate without limiting the invention an embodiment of R⁵ is hydrogen; another embodiment of R⁵ is C₁₋₆-alkyl, such as methyl; a further embodiment of R⁵ is halogen, such as chloro, or fluoro.

In a further embodiment of the compound of formula I R⁶ is selected from hydrogen, halogen, C₁₋₆-alk(en/yn)yl, halo-C₁₋₆-alk(en/yn)yl. Typically, R⁶ is selected from hydrogen, halogen, C₁₋₆-alkyl, halo-C₁₋₆-alkyl. To further illustrate without limiting the invention an embodiment of R⁶ is hydrogen; another embodiment of R⁶ is halogen, such as fluoro.

In a further embodiment of the compound of formula I, R⁷ is selected from hydrogen, halogen, C₁₋₆-alk(en/yn)yl, halo-C₁₋₆-alk(en/yn)yl. Typically, R⁷ is selected from hydrogen, halogen, C₁₋₆-alkyl, halo-C₁₋₆-alkyl. To further illustrate without limiting the invention an embodiment of R⁷ is hydrogen; another embodiment of R⁷ is halogen, such as fluoro.

In a further embodiment of the compound of formula I, R⁸ is selected from hydrogen, halogen, C₁₋₆-alk(en/yn)yl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk-(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom. In a further embodiment of the compound of formula I R⁸ is selected from hydrogen, halogen, C₁₋₆-alk(en/yn)yl, halo-C₁₋₆-alk(en/yn)yl. In a further embodiment R⁸ is NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, such as hydrogen, cyanomethyl, C₁₋₆-alk(en/yn)yl. In a further embodiment R⁸ is NR^{x}R^{y} wherein R^{x} is NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, and R^{y} is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl. In a further embodiment R⁸ is NR^{x}R^{y} wherein R^{x} and R^{y} together with the nitrogen to which they are attached form a 3-7-membered ring which optionally contains one further heteroatom, such as 1-morpholinyl, 1-piperidinyl, 1-azepinyl, 1-piperazinyl, 1-homopiperazinyl, 1-imidazolyl, 1-pynolidinyl, 1-azetidinyl, 1-pyrrolyl or pyrazolyl, optionally substituted with one or more selected from a C₁₋₆-alk(en/yn)yl, hydroxy, hydroxy-C₁₋₆-alk(en/yn)yl, C₁₋₆-alk(en/yn)yloxy-C₁₋₆-alk(en/yn)yl, e.g. one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. Typically, R⁸ is selected from hydrogen; halogen; cyano; C₁₋₆-alkyl; C₁₋₆-alkyloxy; C₁₋₆-alkylsulfanyl; halo-C₁₋₆-alkyl; NR^{x}R^{y} wherein R^{x} and R^{y} are independently selected from hydrogen, C₁₋₆-alkyl, cyanomethyl; NR^{x}R^{y} wherein R^{y} is selected from hydrogen, or C₁₋₆-alkyl, and R^{x} is NR^{z}R^{w}-C₁₋₆-alk(en/yn)yl wherein R^{z} and R^{w} are independently selected from hydrogen, or C₁₋₆-alkyl; 1-morpholinyl, 1-piperidinyl, 1-azepinyl, 1-piperazinyl, 1-homopiperazinyl, 1-imidazolyl, 1-pyrrolidinyl, 1-azetidinyl, 1-pyrrolyl or pyrazolyl, optionally substituted with one or two selected from hydroxy, hydroxy-C₁₋₆-alkyl, C₁₋₆-alkyloxy-C₁₋₆-alkyl, C₁₋₆-alkyl, in particular one or two selected from hydroxy, methoxy-methyl, methyl. To further illustrate without limiting the invention an embodiment of R⁸ is hydrogen; another embodiment of R⁸ is halogen, such as fluoro, or bromo; a further embodiment of R⁸ is C₁₋₆-alkyl, such as methyl; a further embodiment of R⁸ is halo-C₁₋₆-alkyl, such as CF₃.

In a further embodiment of the compound of formula I, R⁹ is selected from hydrogen, halogen, C₁₋₆-alk(en/yn)yl, halo-C₁₋₆-alk(en/yn)yl. Typically, R⁹ is selected from hydrogen, halogen, C₁₋₆-alkyl, halo-C₁₋₆-alkyl. To further illustrate without limiting the invention an embodiment of R⁹ is hydrogen.

In a further embodiment of the compound of formula I, the dotted line ---- indicates a single bond.

In a further embodiment of the compound of formula I, the dotted line ---- indicates a double bond.

Typically, the compound of formula I has at least one substituent in the phenyl ring(s), selected from any one of R¹-R⁹, which is different from hydrogen, such as 1, 2, 3, or 4 substituents in the phenyl ring(s), selected from any one of R¹-R⁹, which is/are different from hydrogen, and the remaining substituents are hydrogen. Thus, in a further embodiment 1 substituent selected from any one of R¹-R⁹, which is different from hydrogen, is present in either of the two phenyl rings, such as 1 substituent selected from R¹-R⁵, or the substituent is selected from R⁶-R⁹. In a further embodiment 2 substituents selected from R¹-R⁹, which are different from hydrogen, are present in either of the two phenyl rings, such as 1 substituent selected from R¹-R⁵, and the other selected from R⁶-R⁹, or both substituents are selected from R¹-R⁵; in this respect R² and R³ may be taken together to form the heterocycle as defined above. In a further embodiment 3 substituents selected from R¹-R⁹, which are different from hydrogen, are present in either of the two phenyl rings, such as 2 substituents selected from R¹-R⁵, and the last substituent is selected from R⁶-R⁹. In each embodiment, as mentioned the remaining substituents are hydrogen. To illustrate this further without limiting the invention, some typical embodiments are outlined hereafter.

Thus, in a further embodiment of the compound of formula I one substituent is present which is R² as defined above, except hydrogen. In a further embodiment of the compound of formula I one substituent is present which is R³ as defined above, except hydrogen. In a further embodiment of the compound of formula I two substituents are present being R³ and R⁸, wherein R³ and R⁸ are as defined above, except hydrogen. In a further embodiment of the compound of formula I two substituents are present being R³ and R⁶, wherein R³ and R⁶ are as defined above, except hydrogen. In a further embodiment of the compound of formula I two substituents are present being R³ and R⁷, wherein R³ and R⁷ are as defined above, except hydrogen. In a further embodiment of the compound of formula I two substituents are present being R¹ and R³, wherein R¹ and R³ are as defined above, except hydrogen. In a further embodiment of the compound of formula I two substituents are present being R² and R³, wherein R² and R³ are as defined above, except hydrogen, in this respect R² and R³ may be taken together to form the heterocycle as defined above. In a further embodiment of the compound of formula I three substituents are present being R¹, R³ and R⁸, wherein R¹, R³ and R⁸ are as defined above, except hydrogen. In each embodiment, as mentioned above the remaining substituents are hydrogen.

In a further embodiment of the compound of formula I, said compound is selected from
*4-[2-(2,4-Dimethylphenoxy)phenyl]-1,2,3,6-tetrahydropyridine,*
*4-[2-(4-Chlorophenoxy)phenyl]*-*1,2,3,6-tetrahydropyridine,*
*4-[2-(4-Fluoro-*2*-methylphenoxy)phenyl]*-*1,2,3,6-tetrahydropyridine,*
*4-[2-(4-Fluorophenoxy)phenyl]-1,2,3,6-tetrahydropyridine,*
*4-[2-(4-Methylphenoxy)phenyl]-1,2,3,6-tetrahydropyridine,*
*4-[2-(4-Methoxyphenoxy)phenyl]-1,2,3,6-tetrahydropyridine,*
*4-[2-(2,4-Dimethylphenoxy)phenyl]piperidine,*
*4-[2-(4-Chlorophenoxy)phenyl]piperidine,*
*4-[2-(4-Fluoro-2-methylphenoxy)phenyl]piperidine,*
*4-[2-(4-Fluorophenoxy)pltenyl]piperidine,*
*4-[2-(4-Methylphenoxy)phenyl]piperidine,*
*4-[2-(4-Chloro-2-methyl-phenoxy)-phenyl]-piperidine,*
*4-[2-(2-Chloro-4-methyl-phenoxy)-phenyl]-piperidine,*
*4-[2-(2,4-Dichloro-phenoxy)-phenyl]-piperidine,*
*4-[2-(Benzo[b]thiophen-5-yloxy)phenyl]-piperidine,*
*4-[2-(Benzo[1,3]dioxol-5-yloxy)-phenyl]-piperidine,*
*4-[2-(4-Methoxy-2-methyl-phenoxy)-phenyl]piperidine,*
*4-[-2-(3,4-Dichloro-phenoxy)-phenyl]-piperidine,*
*4-[2-(3,4-Dinzethyl-phenoxy)-phenyl]-piperidine,*
*4-[2-(2,3,4,5-Tetranzethyl-phenoxy)-phenyl]-piperidine,*
*4-[2-(4-Trifluoromethyl-phenoxy)-pheinyl]-piperidine,*
*4-[2-(4-Methoxy-phenoxy)-phenyl]-piperidine,*
*4-[2-(2-Chloro-4-methoxy-phenoxy)-phenyl]-piperidine,*
*4-[2-(3,4-Dimethoxy-phenoxy)-phenyl]-piperidine,*
*4-[2-(4-Chloro-3-trifluoromethyl-phenoxy)-phenyl]-piperidine,*
or
a pharmaceutically acceptable salt thereof. Each of these compounds is considered a specific embodiment and may be subject to individual claims.

As mentioned above, most of the tested compounds posses the combined effect of serotonin reuptake inhibition and norepinephrine uptake inhibition, however, a few compounds selected from
*4-[2-(2,4-Dimethylphenoxy)phenyl]-1,2,3,6-tetrahydropyridine,*
*4-[2-(4-Chlorophenoxy)phetzyl]-1, 2,3,6-tetrahydropyridine,*
*4-[2-(4-Fluoro-2-methylphenoxy)phenyl]-1,2,3,6-tetrahydropyridine,*
*4-[2-(4-Fluorophenoxy)phenyl]-1,2,3,6-tetrahydropyridine,*
*4-[2-(4-Methylphenoxy)phenyl]-1,2,3,6-tetrahydropyridine,*
*4-[2-(4-Methoxyphenoxy)phenyl]-1,2,3,6-tetrahydropyridine,*
*4-[2-(3,4-Dimethyl-phenoxy)-phenyl]-piperidine,*
*4-[2-(2,3,4,5-Tetramethyl-phenoxy)-phenyl]-piperidine,*
*4-[2-(3,4-Dimethoxy-phenoxy)-phenyl]-piperidine,*
did show serotonin reuptake inhibition, but did not show norepinephrine uptake inhibition in the test herein.

The present invention also comprises salts of the present compounds, typically, pharmaceutically acceptable salts. Such salts include pharmaceutical acceptable acid addition salts, pharmaceutically acceptable metal salts, ammonium and alkylated ammonium salts. Acid addition salts include salts of inorganic acids as well as organic acids.

Representative examples of suitable inorganic acids include hydrochloric, hydrobromic, hydroiodic, phosphoric, sulfuric, sulfamic, nitric acids and the like. Representative examples of suitable organic acids include formic, acetic, trichloroacetic, trifluoroacetic, propionic, benzoic, cinnamic, citric, fumaric, glycolic, itaconic, lactic, methanesulfonic, maleic, malic, malonic, mandelic, oxalic, picric, pyruvic, salicylic, succinic, methane sulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, theophylline acetic acids, as well as the 8-halotheophyllines, for example 8-bromotheophylline and the like.

Examples of metal salts include lithium, sodium, potassium, magnesium salts and the like.

Examples of ammonium and alkylated ammonium salts include ammonium, methyl-, dimethyl-, trimethyl-, ethyl-, hydroxyethyl-, diethyl-, n-butyl-, sec-butyl-, tert-butyl-, tetramethylammonium salts and the like.

Further, the compounds of this invention may exist in unsolvated as well as in solvated forms with pharmaceutically acceptable solvents such as water, ethanol and the like. In general, the solvated forms are considered equivalent to the unsolvated forms for the purposes of this invention.

The compounds of the present invention may have one or more asymmetric centres and it is intended that any optical isomers (i.e. enantiomers or diastereomers), as separated, pure or partially purified optical isomers and any mixtures thereof including racemic mixtures are included within the scope of the invention.

Racemic forms can be resolved into the optical antipodes by known methods, for example, by separation of diastereomeric salts thereof with an optically active acid, and liberating the optically active amine compound by treatment with a base. Another method for resolving racemates into the optical antipodes is based upon chromatography on an optically active matrix. Racemic compounds of the present invention can also be resolved into their optical antipodes, e.g. by fractional crystallization of d- or 1- (tartrates, mandelates or camphorsulphonate) salts. The compounds of the present invention may also be resolved by the formation of diastereomeric derivatives.

Additional methods for the resolution of optical isomers, known to those skilled in the art, may be used. Such methods include those discussed by J. Jaques, A. Collet and S. Wilen in "Enantiomers, Racemates, and Resolutions", John Wiley and Sons, New York (1981).

Optically active compounds can also be prepared from optically active starting materials, or by stereoselective synthesis.

Furthermore, when a double bond or a fully or partially saturated ring system is present in the molecule geometric isomers may be formed. It is intended that any geometric isomers, as separated, pure or partially purified geometric isomers or mixtures thereof are included within the scope of the invention. Likewise, molecules having a bond with restricted rotation may form geometric isomers. These are also intended to be included within the scope of the present invention.

Furthermore, some of the compounds of the present invention may exist in different tautomeric forms and it is intended that any tautomeric forms that the compounds are able to form are included within the scope of the present invention.

The invention also encompasses active metabolites of the present compounds.

As mentioned above, the compounds of formula I are serotonin reuptake inhibitors, and accordingly may be applicable for the treatment, including prevention, of affective disorders, such as depression, anxiety disorders including general anxiety disorder and panic disorder and obsessive compulsive disorder.

Accordingly, in a further aspect the invention relates to a compound of formula I for use as a medicament.

The present invention also relates to a pharmaceutical composition comprising a compound of formula I and a pharmaceutically acceptable carrier or diluent. The composition may comprise any one of the embodiments of formula I described above.

In an embodiment of the pharmaceutical composition, the compound of formula I is present in an amount of from about 0.001 to about 100 mg/kg body weight per day.

The present invention also relates to use of a compound of formula I for the preparation of a medicament for the treatment of a disease or disorder, wherein a serotonin reuptake inhibitor is beneficial. The medicament may comprise any one of the embodiments of formula I described above.

In particular, the present invention also relates to use of a compound of formula I for the preparation of a medicament for the treatment of affective disorders.

In a further embodiment the present invention also relates to use of a compound of formula I for the preparation of a medicament for the treatment of depression.

In a further embodiment, the present invention also relates to use of a compound of formula I for the preparation of a medicament for the treatment of anxiety disorders.

In a further embodiment, the present invention also relates to use of a compound of formula I for the preparation of a medicament for the treatment of general anxiety disorder.

In a further embodiment, the present invention also relates to use of a compound of formula I for the preparation of a medicament for the treatment of social anxiety disorder.

In a further embodiment, the present invention also relates to use of a compound of formula I for the preparation of a medicament for the treatment of post traumatic stress disorder.

In a further embodiment, the present invention also relates to use of a compound of formula I for the preparation of a medicament for the treatment of obsessive compulsive disorder.

In a further embodiment, the present invention also relates to use of a compound of formula I for the preparation of a medicament for the treatment of panic disorder.

In a further embodiment, the present invention also relates to use of a compound of formula I for the preparation of a medicament for the treatment of panic attacks.

In a further embodiment, the present invention also relates to use of a compound of formula I for the preparation of a medicament for the treatment of specific phobias.

In a further embodiment, the present invention also relates to use of a compound of formula I for the preparation of a medicament for the treatment of social phobia.

In a further embodiment, the present invention also relates to use of a compound of formula I for the preparation of a medicament for the treatment of agoraphobia.

The compounds of the invention can be used in a method for the treatment of a disease or disorder selected from the group consisting of an affective disorder, such as depression, anxiety disorders including general anxiety disorder, social anxiety disorder, post traumatic stress disorder, obsessive compulsive disorder, panic disorder, panic attacks, specific phobias, social phobia and agoraphobia in a living animal body, including a human.

In a further aspect, the present invention relates to a method of preparing a compound of formula I, comprising
a) Deprotection or cleavage from a polymer support of a compound with formula II 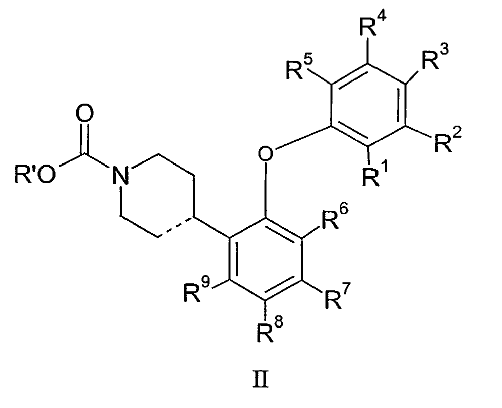 wherein the dotted line, R¹-R⁹ are as previously described, and R' is a *tert*-butyl, methyl, ethyl, allyl or benzyl group or R'OCO is a solid supported carbamate group; or
b) Dehydrating and optionally simultaneously deprotecting a compound of formula III 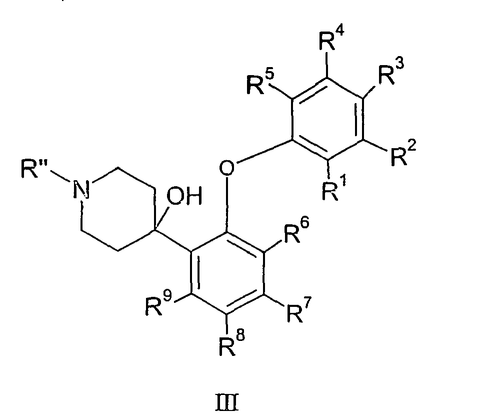 wherein R¹-R⁹ are as previously described, and R" is either a hydrogen atom or a carbamate group R'OCO wherein R' is a *tert*-butyl, methyl, ethyl, allyl or benzyl group or R'OCO is a solid supported carbamate group; or
c) Reduction of the double bond in a compound of formula IV 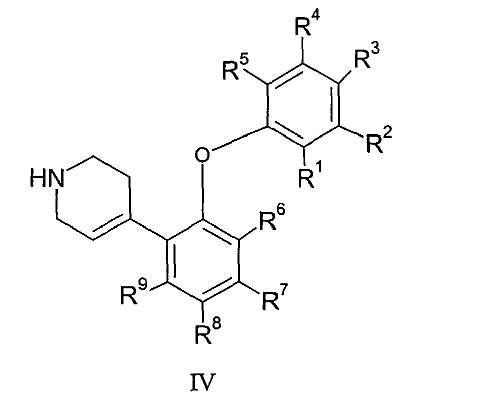
wherein R¹-R⁹ are as previously described.

### Pharmaceutical compositions

The compounds of the invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as capsules, tablets, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, suspensions, syrups and elixirs.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.01 to about 1000 mg, preferably from about 0.05 to about 500 mg, and more preferred from about 0.5 mg to about 200 mg.

For parenteral routes such as intravenous, intrathecal, intramuscular and similar administration, typically doses are in the order of about half the dose employed for oral administration.

The compounds of this invention are generally utilized as the free substance or as a pharmaceutically acceptable salt thereof. One example is an acid addition salt of a compound having the utility of a free base. When a compound of the formula (I) contains a free base such salts are prepared in a conventional manner by treating a solution or suspension of a free base of the formula (I) with a chemical equivalent of a pharmaceutically acceptable acid. Representative examples are mentioned above.

For parenteral administration, solutions of the novel compounds of the formula (I) in sterile aqueous solution, aqueous propylene glycol, aqueous vitamin E or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospho lipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining the novel compounds of the formula (I) and the pharmaceutical acceptable carriers are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

If a solid carrier is used for oral administration, the preparation may be a tablet, placed in a hard gelatine capsule in powder or pellet form or it can be in the form of a troche or lozenge.

The amount of solid carrier will vary widely but will usually be from about 25 mg to about 1 g.

If a liquid carrier is used, the preparation may be in the form of a syrup, emulsion, soft gelatine capsule or sterile injectable liquid such as an aqueous or non-aqueous liquid suspension or solution.

The compounds of the invention are prepared by the following general methods, or as described in the experimental section of this patent:
a) Deprotection or cleavage from a polymer support of a compound with formula II 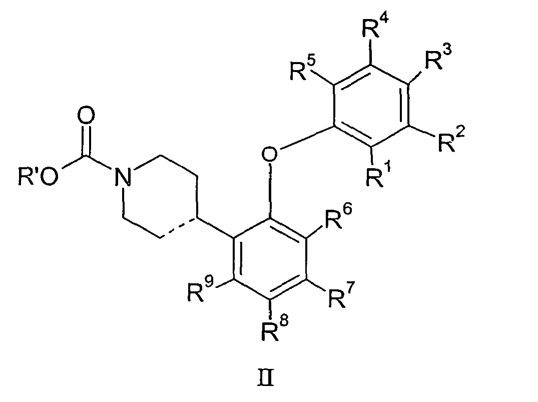 wherein R¹-R⁹ are as previously described, and R' is a *tert*-butyl, methyl, ethyl, allyl or benzyl group or R'OCO is a solid supported carbamate group, such as the Wang resin-based carbamate linker;
b) Dehydrating and optionally simultaneously deprotecting a compound of formula III 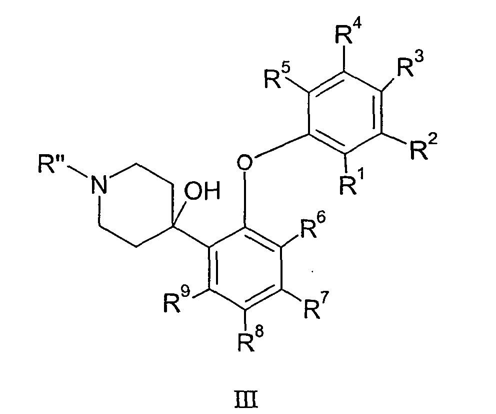 wherein R¹-R⁹ are as previously described, and R" is either a hydrogen atom or a carbamate group R'OCO wherein R' is a *tert*-butyl, methyl, ethyl, allyl or benzyl group or R'OCO is a solid supported carbamate group, such as the Wang resin-based carbamate linker;
c) Reduction of the double bond in a compound of formula IV 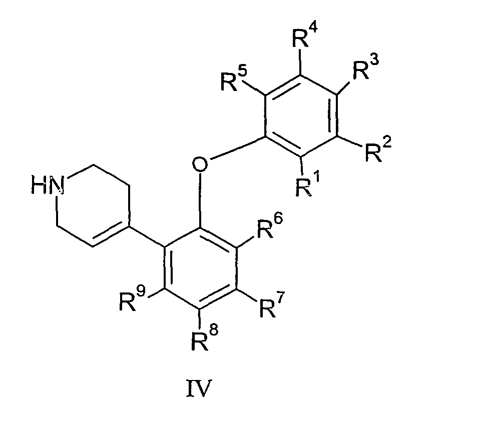 wherein R¹-R⁹ are as previously described.

The deprotection according to method a) was performed by standard techniques, knowledgeable to those skilled in the art and detailed in the textbook Protective Groups in Organic Synthesis T.W.Greene and P.G.M. Wuts, Wiley Interscience, (1991) ISBN 0471623016. The cleavage from a polymer support, such as from the Wang resin-based carbamate linker, according to method a) may be performed according to literature known procedures (Zaragoza Tetrahedron Lett. 1995, 36, 8677-8678 and Conti et al. Tetrahedron Lett. 1997, 38, 2915-2918).

Starting materials of formula II can be prepared by removal of the hydroxy group of compounds of formula III by a number of methods known to the chemist skilled in the art, e.g. by the use of triethylsilane in trifluoro acidic acid and boron trifluoride diethyl etherate (see Encyclopaedia of Reagents for Organic Synthesis, vol 7, Paquette, ed.; John Wiley & Sons, Chichester, 1995, 5122-5123). Starting materials of formula II, which are piperidines, may be prepared by reduction of the double bond of the corresponding tetrahydropyridines by standard hydrogenation procedures, such as e.g. catalytic hydrogenation at low pressure (< 3 atm.) in a Parr apparatus.

The dehydration reaction and optional simultaneous deprotection of a compound of formula III according to method b) was performed in a similar manner as described in Palmer et al J. Med. Chem. 1997, 40, 1982-1989.

Starting materials of formula III were prepared from the corresponding properly substituted 1-bromo-2-phenoxybenzenes of formula VI (wherein R¹-R⁹ are as previously described, and G is a bromine or iodine atom) by metal-halogen exchange followed by addition of an appropriate electrophile of the formula V (wherein R' is as previously described) in a similar manner as described in Palmer et al. J. Med. Chem. 1997, 40,1982-1989.

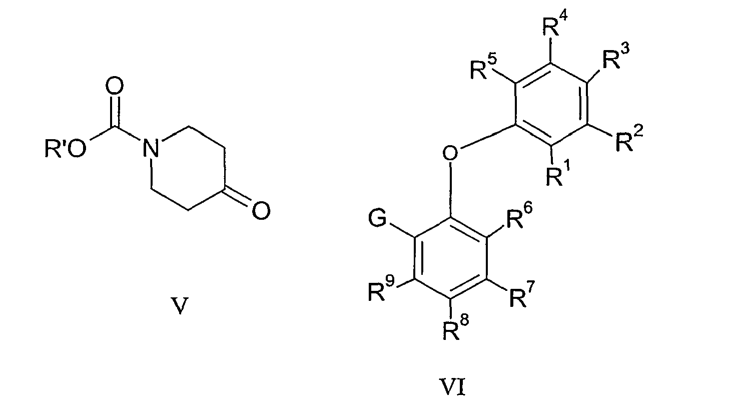

The properly substituted 1-bromo-2-phenoxybenzenes were prepared by reaction of properly substituted phenols (the sodium salt of the phenols were prepared in situ by the use of sodium hydride) with properly substituted 1-bromo-2-fluorobenzenes in dimethyl formamide (DMF) at elevated temperature. The diaryl ethers may also be prepared by various modifications of this method (see e.g. Schmittlinger et al J.Org.Chem. 1993, 58, 3229-3230; Beugelmans et al Tetrahedron Lett. 1994, 35, 5649-5652; Sawyer et al J.Org.Chem. 1998, 63, 6338-6343), under Ullmann conditions or via arylation of phenols with arylboronic acids (Evans et al Tetrahedron Lett 1998, 39, 2937-2940). Phenols and 1-bromo-2-fluorobenzenes are commercially available.

The reduction of the double bond according to method c) is generally performed by catalytic hydrogenation at low pressure (< 3 atm.) in a Parr apparatus. Starting materials of formula IV may be prepared from compounds of formula II by deprotection as described above.

### Examples

Analytical LC-MS data were obtained on a PE Sciex API 150EX instrument equipped with IonSpray source and Shimadzu LC-8A/SLC-10A LC system. Column: 30 X 4.6 mm Waters Symmmetry C18 column with 3.5 µm particle size; Solventsystem: A = water/trifluoroacetic acid (100:0.05) and B = water/acetonitrile/trifluoroacetic acid (5:95:0.03); Method: Linear gradient elution with 90% A to 100% B in 4 min and with a flow rate of 2 mL/min. Purity was determined by integration of the UV (254 nm) and ELSD trace. The retention times (RT) are expressed in minutes.
Preparative LC-MS-purification was performed on the same instrument. Column: 50 X 20 mm YMC ODS-A with 5 µm particle size; Method: Linear gradient elution with 80% A to 100% B in 7 min and with a flow rate of 22.7 mL/min. Fraction collection was performed by split-flow MS detection.
Reactions carried out under microwave conditions were performed in a SmithSynthesizer from Personal Chemistry operating at 2450 MHz.

### Preparation of intermediates

### Preparation of substituted 2-bromo-(substituted-phenoxy)benzenes

### 1-Bromo-2-(2,4-dimethyl-phenoxy)-benzene

A solution 2,4-dimethylphenol (2.4 g) in dry dimethyl formamide (DMF) (10 mL) was added drop wise to a mixture of sodium hydride (1.0 g, 60% in mineral oil) and dry DMF (25 mL), and the resulting mixture was stirred at 100 °C for 30 min. To this mixture was further added 1-bromo-2-fluorobenzene (3.5 g) in dry DMF (5 mL), and the resulting mixture was stirred at 150 °C for 6 hours. The mixture was subsequently poured onto an ice/water mixture, and the aqueous phase was extracted with diethyl ether. The combined organic phase was washed with 2N sodium hydroxide, dried (MgSO₄), filtered and concentrated *in vacuo.* The residue was purified by flash chromatography on silica gel (eluent: heptane) to give the crude product (1.2 g, 70% pure). The crude product was used in the next step without further purification.

The following compounds were prepared in a similar manner:
*1-Bromo-2-(4-chlorophenoxy)-benzene*
*1-Bromo-2-(4-fluoro-2-methylphenoxy)-benzene*
*1-Bromo-2-(4-fluorophenoxy)-benzene*
*1-Bromo-2-(4-methylphenoxy)-benzene*
*1-Bromo-2-(4-methoxyphenoxy)-benzene*

### Preparation of alkyl 4-[2-(substituted-phenoxy)-substituted-phenyl]-4-hydroxypiperidine-1-carboxylates

### tert-Butyl 4-[2-(2, 4-Dimethylphenoxy)phenyl]-4-hydroxypiperidine-1-carbonylate

A solution of 1-bromo-2-(2,4-dimethyl-phenoxy)-benzene (0.7 g) in dry tetrahydrofuran (THF) (3 mL) was added to a solution of nBuLi (1.6 M in hexane, 2 mL) in dry THF (15 mL) at -78°C. The resulting mixture was stirred at -78 °C for 1 hour and subsequently added a solution of *tert-*butyl 4-oxo-piperidine-1-carboxylate (1.0 g) in dry THF (3 mL). The mixture was stirred for 16 hour at room temperature, and then poured onto a saturated solution of ammonium chloride. The aqueous phase was extracted with diethyl ether, and the combined organic phase was washed with water and brine, and subsequently dried (MgSO₄), filtered and concentrated in vacuo. The residue was purified by flash chromatography on silica gel (eluent: heptane/ethyl acetate 4:1) to give the crude product (0.55 g). The crude product was used in the next step without further purification.

The following compounds were prepared in a similar manner:
*Ethyl 4-[2-(2,4-Dimethylphenoxy)phenyl]-4-hydroxy-piperidine-1-carboxylate*
*Ethyl 4-[2-(4-Chlorophenoxy)phenyl]-4-hydroxy-piperidine-1-carboxylate tert-Butyl 4-[2-(4-Fluoro-2-methylphenoxy)phenyl]-4-hydroxy-piperidine-1-carboxylate*
*Ethyl 4-[2-(4-Fluoro-2-methylphenoxy)phenyl]-4-hydroxy-piperidine-1-carboxylate*
*Ethyl 4-[2-(4-Fluorophenoxy)phenyl]-4-hydroxy-piperidine-1-carboxylate tert-Butyl 4-[2-(4-Methylphenoxy)phenyl]-4-hydroxy-piperidine-1-carboxylate*
*Ethyl 4-[2-(4-Methylphenoxy)phenyl]-4-hydroxy-piperidine-1-carboxylate tert-Butyl 4-[2-(4-Methoxyphenoxy)phenyl]-4-hydroxy-piperidine-1-carboxylate*

### Preparation of ethyl 4-(2-methoxy-phenyl)-piperidine-1-carboxylate

To 26 mmol 4-(2-methoxyphenyl)-piperidine (Maybridge) in 100 ml dry dichloromethane were added 28.6 mmol triethylamine and 78 mmol ethylchloroformate at 0 °C. The solution was stirred at room temperature overnight, washed twice with 0.5 M HCl (125 ml) then dried over MgSO₄ and evaporated. The product was sufficiently pure to be used in the following steps.

### Preparation of ethyl 4-(2-hydroxy-phenyl)-piperidine-1-carboxylate

To 24 mmol ethyl 4-(2-methoxy-phenyl)-piperidine-1-carboxylate in 150 ml dry dichloromethane were added 48 mmol BBr₃ at 0 °C. The solution was stirred at room temperature overnight, washed twice with 0.5 M HCl (125 mL) then dried over MgSO₄ and evaporated. The product was sufficiently pure to be used in the following steps.

### Compounds of the invention:

### Preparation of 4-[2-(substituted-phenoxy)-substituted-phenyl]-1,2,3,6-tetrahydropyridines

### 1a, 4-[2-(2,4-Dimethylphenoxy)phenyl]-1,2,3,6-tetrahydropyridine

A mixture of *tert-butyl* 4-[2-(2,4-dimethylphenoxy)phenyl]-4-hydroxy-pipeiidine- 1 - carboxylate (0.5 g) and a mixture of acidic acid and conc. hydrochloride acid (3:1) was boiled under reflux for 16 hours. The mixture was cooled, poured into alkaline water and extracted with ethyl acetate. The combined organic phase was dried (MgSO₄), filtered and concentrated *in vacuo.* The residue was purified by flash chromatography on silica gel (eluent: ethyl acetate/methanol/triethylamine 8:2:1) to give the target compound (11 mg, 3%). LC/MS (m/z) 280 (MH⁺); RT = 2.16; purity (UV, ELSD): 85%, 97%.

The following compounds were prepared in a similar manner:

### 1b, 4-[2-(4-Chlorophenoxy)phenyl]-1,2,3,6-tetrahydropyridine

From ethyl 4-[2-(4-chlorophenoxy)phenyl]-4-hydroxy-piperidine-1-carboxylate. LC/MS (m/z) 286 (MH⁺); RT = 2.10; purity (UV, ELSD): 85%, 95%; yield: 33 mg (6%).

### 1c, 4-[2-(4-Fluoro-2-methylphenoxy)phenyl]-1,2,3,6-tetrahydropyridine

From *tert-*butyl 4-[2-(4-fluoro-2-methylphenoxy)phenyl]-4-hydroxy-piperidine-1-carboxylate. LC/MS (m/z) 284 (MH⁺); RT = 2.08; purity (UV, ELSD): 97%, 99%; yield: 100 mg (21%).

### 1d, 4-[2-(4-Fluorophenoxy)phenyl]-1, 2, 3, 6-tetrahydropyridine

From ethyl 4-[2-(4-fluorophenoxy)phenyl]-4-hydroxy-piperidine-1-carboxylate. LC/MS (m/z) 270 (MH⁺); RT = 1.93; purity (UV, ELSD): 87%, 97%; yield: 45 mg (11%).

### 1e, 4-[2-(4-Methylphenoxy)phenyl]-1,2, 3, 6-tetrahydropyridine

From *tert*-butyl 4-[2-(4-methylphenoxy)phenyl]-4-hydroxy-piperidine-1-carboxylate. LC/MS (m/z) 266 (MH⁺); RT = 2.04; purity (UV, ELSD): 98%, 99%; yield: 250 mg (24%).

### 1f, 4-[2-(4-Methoxyphenoxy)phenyl]-1,2,3,6-tetrahydropyridine

From *tert*-butyl 4-[2-(4-methoxyphenoxy)phenyl]-4-hydroxy-piperidine-1-carboxylate. LC/MS (m/z) 282 (MH⁺); RT = 1.95; purity (UV, ELSD): 79%, 99%; yield: 14.7 mg (19%).

### Preparation of 4-[2-(substituted-phenoxy)-substituted-phenyl]piperidines

### Method A

### 2a, 4-[2-(2,4-Dimethylphenoxy)phenyl]piperidine

A mixture of ethyl 4-[2-(2,4-dimethylphenoxy)phenyl]-4-hydroxy-piperidine-1-carboxylate (0.6 g), dichloromethane (25 mL), triethylsilane (1 mL), trifluoro acidic acid (0.1 mL) and boron trifluoride diethyl etherate (0.2 mL) was stirred at room temperature for 16 hours. The resulting mixture was poured onto alkaline water and subsequently extracted with ethyl acetate. The combined organic phase was dried (MgSO₄) filtered and concentrated *in vacuo* (0.4 g). The residue was dissolved in a mixture of conc. hydrochloric acid and acidic acid (1:3) (25 mL) and boiled under reflux for 16 hours. The mixture was poured onto alkaline water and subsequently extracted with ethyl acetate. The combined organic phase was dried (MgSO₄), filtered and concentrated *in vacuo.* The residue was purified by flash chromatography on silica gel (eluent: ethyl acetate/methanol/triethylamine 8:2:2) to give the target compound (10.6 mg, 3%). LC/MS (m/z) 282 (MH⁺); RT = 2.22; purity (UV, ELSD): 67%, 83%.

The following compounds were prepared in a similar manner:

### 2b, 4-[2-(4-Chlorophenoxy)phenyl]piperidine

From ethyl 4-[2-(4-chlorophenoxy)phenyl]-4-hydroxy-piperidine-1-carboxylate. LC/MS (m/z) 288 (MH⁺); RT = 2.1; purity (UV, ELSD): 96%, 97%; yield: 41 mg (7%).

### 2c, 4-[2-(4-Fluoro-2-methylphenoxy)phenyl]piperidine

From ethyl 4-[2-(4-fluoro-2-methylphenoxy)phenyl]-4-hydroxy-piperidine-1-carboxylate. LC/MS (m/z) 286 (MH⁺); RT = 2.1; purity (UV, ELSD): 89%, 99%; yield: 51 mg (8%).

### 2d, 4-[2-(4-Fluorophenoxy)phenyl]piperidine

From ethyl 4-[2-(4-fluorophenoxy)phenyl]-4-hydroxy-piperidine-1-carboxylate. LC/MS (m/z) 272 (MH⁺); RT = 1.97; purity (UV, ELSD): 91%, 99%; yield: 7 mg (5%).

### 2e, 4-[2-(4-Methylphenoxy)phenyl]piperidine

From ethyl 4-[2-(4-methylphenoxy)phenyl]-4-hydroxy-piperidine-1-carboxylate. LC/MS (m/z) 268 (MH⁺); RT = 2.12; purity (UV, ELSD): 88%, 93%; yield: 8 mg (1%).

### Method B

Ethyl 4-(2-Hydroxy-phenyl)-piperidine-1-carboxylate (0.1 mmol) was combined in 0.5 mL 1-methyl-pyrrolidin-2-one with 0.12 mmol of an appropriate aryl bromide or iodide. CuI catalyst (0.037 mmol) was added and the vial sealed before it was heated for 1 hour in a microwave oven at 220 °C. The solvent was removed from the samples, and a solution of KOH in water (3.7 mmol), dioxane and ethanol (99,9%) were added, and the mixture was heated at 130 °C for 1 hour in the microwave oven. The samples were then added water and solid NaCl and then subsequently extracted with ethyl acetate. The organic phase was evaporated and the crude product purified by preparative LC-MS. The isolated products were subjected to SCX columns and the free amines submitted for testing as DMSO solutions. The following compounds were prepared by this method and measured molecular mass, measured HPLC-retention time (RT, min) and UV- and ELSD-purities (%) is described in **Table 1**.
**3a,** 4-[2-(4-Chloro-2- methyl-phenoxy)-phenyl]-piperidine
**3b,** 4-[2-(3-Chloro-2- methyl-phenoxy)-phenyl]-piperidine
**3c,** 4-[2-(2-Chloro-4-methyl-phenoxy)-phenyl]-piperidine
**3d,** 4-[2-(2,4-Dichloro-phenoxy)-phenyl]-piperidine
**3e,** 4-[2-(Benzo[1,3]dioxol-5-yloxy)-phenyl]-piperidine
**3f,** 4-[2-(4-Methoxy-2-methyl-phenoxy)-phenyl]-piperidine
**3g,** 4-[2-(3,4-Dichloro-phenoxy)-phenyl]-piperidine
**3h,** 4-[2-(3,4-Dimethyl-phenoxy)-phenyl]-piperidine
**3i,** 4-[2-(2,3,4,5-Tetramethyl-phenoxy)-phenyl]-piperidine
**3j,** 4-[2-(4-Trifluoromethyl-phenoxy)-phenyl]-piperidine
**3k,** 4-[2-(4-Methoxy-phenoxy)-phenyl]-piperidine
**3l,** 4-[2-(2-Chloro-4-methoxy-phenoxy)-phenyl]-piperidine
**3m,** 4-[2-(3,4-Dimethoxy-phenoxy)-phenyl]-piperidine
**3n,** 4-[2-(4-Chloro-3-trifluoromethyl-phenoxy)-phenyl]-piperidine

**Table 1. Measured molecular mass, measured HPLC-retention time (RT, min) and UV- and ELSD-purities (%).**

| **compound** | **M+H⁺** | **RT min.** | **UV-purity (%)** | **ELSDpurity (%)** |
|---|---|---|---|---|
| **3a** | | 2,1 | 88.6% | 99.1% |
| **3b** | 302,1 | 2,19 | 92,43 | 92,56 |
| **3c** | 302,1 | 2,18 | 88,64 | 94,63 |
| **3d** | 321,9 | 2,23 | 87,08 | 92,55 |
| **3e** | 297,9 | 1,90 | 92,96 | 89,84 |
| **3f** | 298,3 | 2,02 | 97,69 | 96,69 |
| **3g** | 322,1 | 2,21 | 97,55 | 85,38 |
| **3h** | 282,2 | 2,18 | 96,25 | 91,84 |
| **3i** | 310,2 | 2,42 | 83,51 | 95,12 |
| **3j** | 321,9 | 2,19 | 96,8 | 99,05 |
| **3k** | 284,3 | 1,92 | 99,14 | 97,5 |
| **3l** | 318,1 | 2,07 | 73,27 | 85,01 |
| **3m** | 314,1 | 1,78 | 97,94 | 99,05 |
| **3n** | 356,2 | 2,34 | 98,68 | 88,11 |

### Measurements of [³H]-5-HT uptake into rat cortical synaptosomes.

Whole brains from male Wistar rats (125-225 g), excluding cerebellum, are homogenized in 0.32 M sucrose supplemented with 1mM nialamid with a glass/teflon homogenizer. The homogenate is centrifuged at 600 x g for 10 min at 4 °C. The pellet is discarded and the supernatant is centrifuged at 20.000 x g for 55 min. The final pellet is homogenized (20 sec) in this assay buffer (0.5 mg original tissue/well). Test compounds (or buffer) and 10 nM [³H]-5-HT are added to 96 well plates and shaken briefly. Composition of assay buffer: 123 mM NaCl, 4.82 mM KCl, 0.973 mM CaCl₂, 1.12 mM MgSO₄, 12.66 mM Na₂HPO₄, 2.97 mM NaH₂PO₄, 0.162 mM EDTA, 10 mM glucose and 1 mM ascorbic acid. Buffer is oxygenated with 95% 0₂/5% C0₂ for 10 min at 37 °C and pH is adjusted 7.4. The incubation is started by adding tissue to a final assay volume of 0.2 mL. After 15 min incubation with radioligand at 37 °C, samples are filtered directly on Unifilter GF/C glass fiber filters (soaked for 1 hour in 0.1% polyethylenimine) under vacuum and immediately washed with 3 x 0.2 ml assay buffer. Non-specific uptake is determined using citalopram (10 µM final concentration). Citalopram is included as reference in all experiments as dose-response curve.

### Measurements of [³H]noradrenaline uptake into rat cortical synaptosomes.

Fresh cortex from male Wistar rats (125-225 g) are homogenized in 0.4M sucrose with a glass/teflon homogenizer. The homogenate is centrifuged at 600 x g for 10 min at 4 °C. The pellet is discarded and the supernatant is centrifuged at 20.000 x g for 55 min. The final pellet is homogenized (20 sec) in this assay buffer (6 mg original tissue/mL = 4 mg/well). Test compounds (or buffer) and 10 nM [³H]-noradrenaline are added to deep 96 well plates and shaken briefly. Composition of assay buffer: 123 mM NaCl, 4.82 mM KCl, 0.973 mM CaCl₂, 1.12 mM MgSO₄, 12.66 mM Na₂HPO₄, 2.97 mM NaH₂PO₄, 0.162 mM EDTA, 10 mM glucose and 1 mM ascorbic acid. Buffer is oxygenated with 95% 0₂/5% CO₂ for 10 min at 37 °C and pH is adjusted 7.4. The incubation is started by adding tissue to a final assay volume of 1 ml. After 15 min incubation with radioligand at 37 °C, samples are filtered directly on Unifilter GF/C glass fiber filters (soaked for 1 hour in 0.1% polyethylenimine) under vacuum and immediately washed with 3 x 1 mL assay buffer. Non-specific uptake is determined using talsupram (10 µM final concentration). Duloxetine is included as reference in all experiments as dose-response curve.

Results of the experiments showed that the tested compounds of the invention inhibit the norepinephrine and serotonine reuptake with IC₅₀ below 200 nM.

## Claims

1. A compound represented by the general formula I 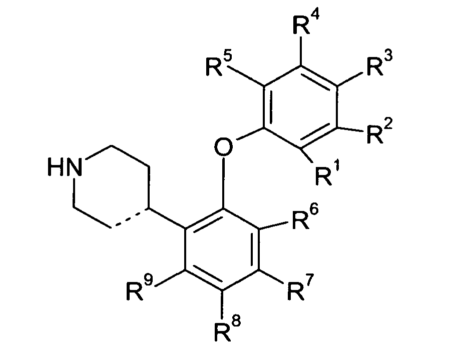 Wherein
the dotted line ---- indicates a single bond or a double bond;
R¹, R², R³, R⁴, R⁵ are independently selected from hydrogen, halogen, cyano, C₁-₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; or
R² and R³ together form a heterocycle fused to the phenyl ring selected from 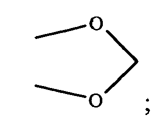 and R¹, R⁴, R⁵ are as defined above;
R⁶, R⁷, R⁸, R⁹ are independently selected from hydrogen, halogen, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, or NR^{x}R^{y} wherein R^{x} and R^{y} are independently selected from hydrogen, C₁₋₆-alk(enlyn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom;
provided that at least one of R¹, R², R³, R⁴, R⁵, R⁶, R⁷, R⁸, and R⁹ is different from hydrogen;
or a salt thereof.

2. The compound of claim 1, wherein R¹ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}k^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; typically, R¹ is selected from hydrogen, C₁₋₆-alkyl, or halogen.

3. The compound of any one of claims 1-2, wherein R² is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl; typically, R² is selected from hydrogen, C₁₋₆-alkoxy, halo-C₁₋₆-alkyl, C₁₋₆-alkyl, or halogen; more typically, R² is selected from hydrogen, or C₁₋₆-alkoxy.

4. The compound of any one of claims 1-3, wherein R³ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆-alk(en/yn)yl; typically, R³ is selected from hydrogen, C₁₋₆-alkyl, C₁₋₆-alkoxy, halogen, or halo-C₁₋₆-alkyl; more typically, R³ is selected from hydrogen, C₁₋₆alkyl, C₁₋₆-alkoxy, or halogen.

5. The compound of any one of claims 1-2, wherein R² and R³ together form a heterocycle fused to the phenyl ring selected from

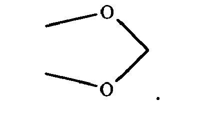

6. The compound of any one of claims 1-5 wherein R⁴ is selected from hydrogen, halogen, cyano, C₁₋₆-alk(en/yn)yl, C₁₋₆-alk(en/yn)yloxy, C₁₋₆-alk(en/yn)ylsulfanyl, halo-C₁₋₆alk(en/yn)yl; typically, R⁴ is selected from hydrogen, C₁₋₆-alkoxy, halo-C₁₋₆-alkyl, C₁₋₆-alkyl, or halogen; more typically, R⁴ is selected from hydrogen, or C₁₋₆-alkoxy.

7. The compound of any one of claims 1-6 wherein R⁵ is selected from hydrogen, halogen, cyano, C₁₋₆alk(en/yn)yl, C₁₋₆-alk(en/yn)yloxy, C₁₋₆-alk(en/yn)ylsulfanyl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; typically, R⁵ is selected from hydrogen, C₁₋₆-alkyl, or halogen.

8. The compound of any one of claims 1-7 wherein R⁶ is selected from hydrogen, halogen, C₁₋₆-alk(en/yn)yl, halo-C₁₋₆-alk(en/yn)yl; typically, R⁶ is selected from hydrogen, or halogen.

9. The compound of any one of claims 1-8 wherein R⁷ is selected from hydrogen, halogen, C₁₋₆-alk(en/yn)yl, halo-C₁₋₆-alk(en/yn)yl; typically, R⁷ is selected from hydrogen, or halogen.

10. The compound of any one of claims 1-9 wherein R⁸ is selected from hydrogen, halogen, C₁₋₆-alk(en/yn)yl, halo-C₁₋₆-alk(en/yn)yl, or NR^{x}R^{y} wherein R^{x} and R^{y} are independently selected from hydrogen, C₁₋₆-alk(en/yn)yl, cyano-C₁₋₆-alk(en/yn)yl, C₃₋₈-cycloalk(en)yl, C₃₋₈-cycloalk(en)yl-C₁₋₆-alk(en/yn)yl, or NR^{z}R^{w}-C₁₋₆-alk(en/yn)yl, wherein R^{z} and R^{w} are independently selected from hydrogen, C₁₋₆-alk(en/yn)yl, C₃₋₈-cycloalk(en)yl, or C₃₋₈-cycloalk(en)yl-C₁₋₆-alk(en/yn)yl, provided that if one of R^{x} and R^{y} is NR^{z}R^{w}-C₁₋₆-alk(en/yn)yl then the other is selected from hydrogen, C₁₋₆-alk(en/yn)yl, cyano-C₁₋₆-alk(en/yn)yl, C₃₋₈-cycloalk(en)yl, or C₃₋₈-cycloalk(en)yl-C₁₋₆-alk(en/yn)yl; or R^{x} and R^{y} together with the nitrogen to which they are attached form a 3-7-membered ring which optionally contains one further heteroatom; typically, R⁸ is selected from hydrogen, halo-C₁₋₆-alkyl, C₁₋₆-alkyl, or halogen.

11. The compound of any one of claims 1-10 wherein R⁹ is selected from hydrogen, halogen, C₁₋₆-alk(en/yn)yl, halo-C₁₋₆-alk(en/yn)yl; typically, R⁹ is selected from hydrogen.

12. The compound of any one of claims 1-11 wherein the dotted line ---- indicates a single bond.

13. The compound of any one of claims 1-11 wherein the dotted line ---- indicates a double bond.

14. The compound of any one of claims 1-13 wherein the compound of formula I has 1-4 substituents in the phenyl ring(s), selected from any one of R¹-R⁹, which are different from hydrogen, and the remaining substituents are hydrogen.

15. The compound of claim 1, said compound being
4-[2-(2,4-Dimethylphenoxy)phenyl]-1,2,3,6-tetrahydropyridine,
4-[2-(4-Chlorophenoxy)phenyl]-1,2,3,6-tetrahydropyridine,
4-[2-(4-Fluoro-2-methylphenoxy)phenyl]-1,2,3,6-tetrahydropyridine,
4-[2-(4-Fluorophenoxy)phenyl]-1,2,3,6-tetrahydropyridine,
4-[2-(4-Methylphenoxy)phenyl]-1,2,3,6-tetrahydropyridine,
4-[2-(4-Methoxyphenoxy)phenyl]-1,2,3,6-tetrahydropyridine,
4-[2-(2,4-Dimethylphenoxy)phenyl]piperidine,
4-[2-(4-Chlorophenoxy)phenyl]piperidine,
4-[2-(4-Fluoro-2-methylphenoxy)phenyl]piperidine,
4-[2-(4-Fluorophenoxy)phenyl]piperidine,
4-[2-(4-Methylphenoxy)phenyl]piperidine,
4-[2-(4-Chloro-2- methyl-phenoxy)-phenyl]-piperidine
4-[2-(3-Chloro-2- methyl-phenoxy)-phenyl]-piperidine
4-[2-(2-Chloro-4-methyl-phenoxy)-phenyl]-piperidine
4-[2-(2,4-Dichloro-phenoxy)-phenyl]-piperidine
4-[2-(Benzo[1,3]dioxo1-5-yloxy)-phenyl]-piperidine,
4-[2-(4-Methoxy-2-methyl-phenoxy)-phenyl]-piperidine,
4-[2-(3,4-Dichloro-phenoxy)-phenyl]-piperidine,
4-[2-(3,4-Dimethyl-phenoxy)-phenyl]-piperidine,
4-[2-(2,3,4,5-Tetramethyl-phenoxy)-phenyl]-piperidine,
4-[2-(4-Trifluoromethyl-phenoxy)-phenyl]-piperidine,
4-[2-(4-Methoxy-phenoxy)-phenyl]-piperidine,
4-[2-(2-Chloro-4-methoxy-phenoxy)-phenyl]-piperidine,
4-[2-(3,4-Dimethoxy-phenoxy)-phenyl]-piperidine, and
4-[2-(4-Chloro-3-trifluoromethyl-phenoxy)-phenyl]-piperidine;
or a pharmaceutically acceptable salt thereof.

16. A pharmaceutical composition comprising a compound of any one of claims 1-15 or a pharmaceutically acceptable acid addition salt thereof and at least one pharmaceutically acceptable carrier or diluent.

17. The use of a compound of any one of claims 1 to 15 or a pharmaceutically acceptable acid addition salt thereof for the preparation of a medicament for the treatment of affective disorders, such as depression, anxiety disorders including general anxiety disorder, social anxiety disorder, post traumatic stress disorder, obsessive compulsive disorder, panic disorder, panic attacks, specific phobias, social phobia and agoraphobia.

18. A compound of any one of claims 1-15 for use as a medicament.

## Patentansprüche

1. Verbindung, dargestellt durch die allgemeine Formel I 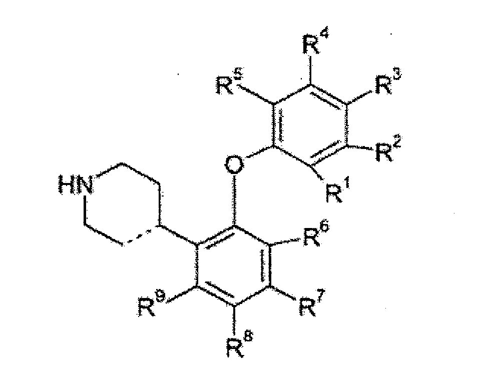 worin die gestrichelte Linie ---- eine Einfachbindung oder eine Doppelbindung bezeichnet;
R¹, R², R³, R⁴, R⁵ unabhängig voneinander aus Wasserstoff, Halogen, Cyano, C₁₋₆-Alk(en/in)yl, C₁₋₆-Alk(en/in)yloxy, C₁₋₆-Alk(en/in)ylsulfanyl, Hydroxy, Hydroxy-C₁₋₆-alk(en/in)yl, Halogen-C₁₋₆-alk(en/in)yl, Halogen-C₁₋₆-alk(en/in)yloxy oder aus NR^{x}R^{y} ausgewählt sind, worin R^{x} und R^{y} unabhängig voneinander aus Wasserstoff, C₁₋₆-Alk(en/in)yl, Cyano-C₁₋₆-alk(en/in)yl C₃₋₈-Cycloalk(en)yl,
C₃₋₈-Cycloalk(en)yl-C₁₋₆-alk(en/in)yl oder aus NR^{z}R^{w}-C₁₋₆-Alk(en/in)yl ausgewählt sind, worin R^{z} und R^{w} unabhängig voneinander aus Wasserstoff, C₁₋₆-Alk(en/in)yl, C₃₋₈-Cycloalk(en)yl oder
C₃₋₈-Cycloalk(en)yl-C₁₋₆-alk(en/in)yl ausgewählt sind; oder R^{x} und R^{y} zusammen mit dem Stickstoff, mit dem sie verbunden sind, einen
3-7-gliedrigen Ring bilden, der gegebenenfalls ein weiteres Heteroatom enthält; oder
R² und R³ zusammen einen Heterozyklus bilden, der an den Phenylring ankondensiert ist, ausgewählt aus 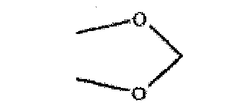 und R¹, R⁴, R⁵ wie oben definiert sind;
R⁶, R⁷, R⁸, R⁹ unabhängig voneinander aus Wasserstof, Halogen, C₁₋₆-Alk(en/in)yl, C₁₋₆-Alk(en/in)yloxy, C₁₋₆-Alk(en/in)ylsulfanyl, Hydroxy, Hydroxy-C₁₋₆-Alk(en/in)yl, Halogen-C₁₋₆-alk(en/in)yl, Halogen-C₁₋₆-alk(en/in)yloxy oder aus NR^{x}R^{y} ausgewählt sind, worin R^{x} und R^{y} unabhängig voneinander aus Wasserstoff, C₁₋₆-Alk(en/in)yl, Cyano-C₁₋₆-alk(enlin) yl, C₃₋₈-Cycloalk(en) yl, C₃₋₈-Cycloalk(en)yl-C₁₋₆-alk(en/in)yl oder aus NR^{z}R^{w}-C₁₋₆-Alk(en/in)yl ausgewählt sind, worin R^{z} und R^{w} unabhängig voneinander aus Wasserstoff, C₁₋₆-Alk(en/in)yl, C₃₋₈-Cycloalk(en)yl oder C₃₋₈-Cycloalk(en)yl-C₁₋₆-alk(en/in)yl ausgewählt sind; oder R^{x} und R^{y} zusammen mit dem stickstoff, mit dem sie verbunden sind, einen 3-7-gliedrigen Ring bilden, der gegebenenfalls ein weiteres Heteroatom enthält;
mit der Maßgabe, dass zumindest eines von R¹, R², R³, R⁴, R⁵, R⁶, R⁷, R⁸ und R⁹ von Wasserstoff verschieden ist;
oder ein Salz davon.

2. Verbindung nach Anspruch 1, worin R¹ aus Wasserstoff, Halogen, Cyano, C₁₋₆-Alk(en/in)yl, C₁₋₆-Alk(en/in) yloxy, C₁₋₆ Alk(en/in)ylsulfanyl, Halogen-C₁₋₆-Alk(en/in)yl oder aus NR^{x}R^{y} ausgewählt ist, worin R^{x} und R^{y} unabhängig voneinander aus Wasserstoff, C₁₋₆-Alk(en/in)yl, Cyano-C₁₋₆-alk (en/in) yl, C₃₋₈-Cycloalk(en)yl, C₃₋₈-Cycloalk(en)yl-C₁₋₆-alk(en/in)yl oder aus NR^{z}R^{w}-C₁₋₆-Alk(en/in)yl ausgewählt sind, worin R^{z} und R^{w} unabhängig voneinander aus Wasserstoff, C₁₋₆-Alk(en/in)yl, C₃₋₈-Cycloalk(en)yl oder C₃₋₈-Cykloalk(en)yl-C₁₋₆-alk(en/in)yl ausgewählt sind, mit der Maßgabe, wenn eines von R^{x} und R^{y} NR^{z}R^{w}-C₁₋₆-Alk(en/in)yl ist, dann das andere aus Wasserstoff, C₁₋₆-Alk(en/in)yl, Cyano-C₁₋₆-alk(en/in)yl, C₃₋₈-Cyloalk(en)yl oder C₃₋₈-Cycloalk(en)yl-C₁₋₆-alk(en/in)yl ausgewählt ist; oder R^{x} und R^{y} zusammen mit dem Stickstoff, mit dem sie verbunden sind, einen 3-7-gliedrigen Ring bilden, der gegebenenfalls ein weiteres Heteroatom enthält; wobei typischerweise R¹ aus Wasserstoff, C₁₋₆-Alkyl oder Halogen ausgewählt ist.

3. Verbindung nach einem der Ansprüche 1-2, worin R² aus Wasserstoff, Halogen, Cyano, C₁₋₆-Alk(en/in)yl, C₁₋₆-Alk(en/in)yloxy, C₁₋₆-Alk(en/in)ylsulfanyl, Halogen-C₁₋₆-alk(en/in)yl ausgewählt ist; typischerweise R² aus Wasserstoff, C₁₋₆-Alkoxy; Halogen-C₁₋₆-alkyl, C₁₋₆-Alkyl oder Halogen ausgewählt ist; R² noch typischer aus Wasserstoff oder C₁₋₆-Alkoxy ausgewählt ist.

4. Verbindung nach einem der Ansprüche 1-3, worin R³ aus Wasserstoff, Halogen, Cyano, C₁₋₆-Alk(en/in)yl, C₁₋₆-Alk(en/in)yloxy, C₁₋₆-Alk(en/in)ylsulfanyl, Halogen-C₁₋₆-alk(en/in)yl ausgewählt ist; typischerweise R³ aus Wasserstoff, C₁₋₆-Alkyl C₁₋₆-Alkoxy, Halogen oder Halogen-C₁₋₆-alkyl ausgewählt ist; R³ noch typischer aus Wasserstoff, C₁₋₆-Alkyl, C₁₋₆-Alkoxy oder Halogen ausgewählt ist.

5. Verbindung nach einem der Ansprüche 1-2, worin R² und R³ zusammen einen Heterozyklus bilden, der an den Phenylring ankondensiert ist, ausgewählt aus

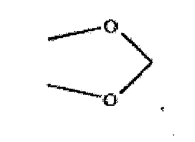

6. Verbindung nach einem der Ansprüche 1-5, worin R⁴ aus Wasserstoff, Halogen, Cyano, C₁₋₆-Alk(en/in)yl, C₁₋₆-Alk(en/in)yloxy, C₁₋₆-Alk(en/in)ylsulfanyl, Halogen-C₁₋₆-Alk(en/in)yl ausgewählt ist; typischerweise R⁴ aus Wasserstoff, C₁₋₆-Alkoxy; Halogen-C₁-₆-alkyl, C₁₋₆-Alkyl oder Halogen ausgewählt ist; noch typischer R⁴ aus Wasserstoff oder C₁₋₆-Alkoxy ausgewählt ist.

7. Verbindung nach einem der Ansprüche 1-6, worin R⁵ aus Wasserstoff, Halogen, Cyano, C₁₋₆-Alk(en/in)yl, C₁₋₆-Alk(en/in)yloxy, C₁₋₆-Alk(en/in)ylsulfanyl, Halogen-C₁₋₆-alk(en/in)yl oder aus NR^{x}R^{y} ausgewählt ist, worin R^{x} und R^{y} unabhängig voneinander aus Wasserstoff, C₁₋₆-Alk(enlin) yl, Cyano-C₁₋₆-alk(enlin) yl, C₃₋₈-Cycloalk(en) yl, G₃₋₈-Cycloalk(en)yl-C₁₋₆-alk(en/in)yl oder aus NR^{z}R^{w}-C₁₋₆-alk(en/in)yl ausgewählt sind, worin R^{z} und R^{w} unabhängig voneinander aus Wasserstoff, C₁₋₆-Alk(en/in)yl, C₃₋₈-Cycloalk(en)yl oder C₃₋₈-Cycloalk(en)yl-C₁₋₆-Alk(en/in)yl ausgewählt sind, mit der Maßgabe, wenn eines von R^{x} und R^{y} NR^{z}R^{w}-C₁₋₆-Alk(en/in)yl ist, dann das andere aus Wasserstoff, C₁₋₆-Alk(en/in)yl, Cyano-C₁₋₆-alk(en/in)yl, C₃₋₈-Cycloalk(en)yl oder C₃₋₈-Cycloalk(en)yl-C₁₋₆-alk(en/in)yl ausgewählt ist; oder R^{x} und R^{y} zusammen mit dem Stickstoff, mit dem sie verbunden sind, einen 3-7-gliedrigen Ring bilden, der gegebenenfalls ein weiteres Heteroatom enthält; typischerweise R⁵ aus Wasserstoff, C₁₋₆-Alkyl oder Halogen ausgewählt ist.

8. Verbindung nach einem der Ansprüche 1-7, worin R⁶ aus Wasserstoff, Halogen, C₁₋₆-Alk(en/in)yl, Halogen-C₁₋₆-Alk(en/in)yl ausgewählt ist; typischerweise R⁶ aus Wasserstoff oder Halogen ausgewählt ist.

9. Verbindung nach einem der Ansprüche 1-8, worin R⁷ aus Wasserstoff, Halogen, C₁₋₆-Alk(en/in)yl, Halogen-C₁₋₆-Alk(en/in)yl ausgewählt ist; typischerweise R⁷ aus Wasserstoff oder Halogen ausgewählt ist.

10. Verbindung nach einem der Ansprüche 1-9, worin R⁸ aus Wasserstoff, Halogen, C₁₋₆-Alk(en/in)yl Halogen-C₁₋₆-Alk(en/in)yl oder aus NR^{x}R^{y} ausgewählt ist, worin R^{x} und R^{y} unabhängig voneinander aus Wasserstoff, C₁₋₆-Alk(en/in)yl, Cyano-C₁₋₆-alk(en/in)yl, C₃₋₈-Cycloalk(en)yl, C₃₋₈-Cycloalk(en)yl-C₁₋₆-alk(en/in)yl oder aus NR^{z}R^{w}-C₁₋₆-Alk(en/in)yl ausgewählt sind, worin R^{z} und R^{w} unabhängig voneinander aus Wasserstoff, C₁₋₆-Alk(en/in)yl, C₃₋₈-Cycloalk(en)yl oder C₃₋₈-Cycloalk(en)yl-C₁₋₆-alk(en/in)yl ausgewählt sind, mit der Maßgabe, wenn eines von R^{x} und R^{y}NR^{z}R^{w}-C₁₋₆-Alk(en/in)yl ist, dann das andere aus Wasserstoff, C₁₋₆-Alk(en/in)yl, Cyano-C₁₋₆-Alk(en/in)yl, C₃₋₈-Cycloalk(en)yl oder C₃₋₈-Cycloalk(en)yl-C₁₋₆-alk(en/in)yl ausgewählt ist; oder R^{x} und R^{y} zusammen mit dem Stickstoff, mit dem sie verbunden sind, einen 3-7-gliedrigen Ring bilden, der gegebenenfalls ein weiteres Heteroatom enthält; typischerweise R⁸ aus Wasserstoff, Halogen-C₁₋₆-alkyl, C₁₋₆-Alkyl oder Halogen ausgewählt ist.

11. Verbindung nach einem der Ansprüche 1-10, worin R⁹ aus Wasserstoff, Halogen, C₁₋₆-Alk(en/in)yl, Halogen-C₁₋₆-Alk(en/in)yl ausgewählt ist; typischerweise R⁹ aus Wasserstoff ausgewählt ist.

12. Verbindung nach einem der Ansprüche 1-11, worin die gestrichelte Linie ---- eine Einfachbindung bezeichnet.

13. Verbindung nach einem der Ansprüche 1-11, worin die gestrichelte Linie ---- eine Doppelbindung bezeichnet.

14. Verbindung nach einem der Ansprüche 1-13, worin die Verbindung der Formel I 1-4 Substituenten in dem Phenylring/den Phenylringen aufweist, ausgewählt aus einem von R¹-R⁹, welche von Wasserstoff verschieden sind, und die verbleibenden Substituenten Wasserstoff sind.

15. Verbindung nach Anspruch 1, wobei die Verbindung
4-[2-(2,9-Dimethylphenoxy)phenyl]-1,2,3,6-tetrahydropyridin,
4-[2-(4-Chlorphenoxy)phenyl]-l,2,3,6-tetrahydropyridin,
4-[2-(4-Fluor-2-methylphenoxy)phenyl]-1,2,3,6-tetrahydropyridin,
9-[2-(4-Fluorphenoxy)phenyl]-1,2,3,&-tetrahydropyridin,
4-[2-(4-Methylphenoxy)phenyl]-1,2,3,6-tetrahydropyridin,
4-[2-(4-Methoxyphenoxy)phenyl]-1,2,3,6-tetrahydxopyridin,
4-[2-(2,4-Dimethylphenoxy)phenyl]piperidin,
4-[2-(4-Chlorphenoxy)phenyl]piperidin,
9-[2-(4-Fluor-2-methylphenoxy)phenyl]piperidin,
4-[2-(4-Fluorphenoxy)phenyl]piperidin,
4-[2-(4-Methylphenoxy)phenyl]piperidin,
4-[2-(4-Chlor-2-methyl-phenoxy)-phenyl]-piperidin,
4-[2-(3-Ghlor-2-methyl-phenoxy)-phenyl]-piperidin,
4-[2-(2-Chlor-4-methyl-phenoxy)-phenyl]-piperidin,
4-[2-(2,4-Dichlor-phenoxy)-phenyl]-piperidin,
4-[2-(Benzo[1,3]dioxol-5-yloxy)-phenyl]-piperidin,
4-[2-(4-Methoxy-2-methyl-phenoxy)-phenyl]-piperidin,
4-[2-(3,4-Dichlor-phenoxy)-phenyl]-piperidin,
4-[2-(3,4-Dimethyl-phenoxy)-phenyl]-piperidin,
4-[2-(2,3,4,5-Tetramethyl-phenoxy)-phenyl]-piperidin,
4-[2-(4-Trifluormethyl-phenoxy)-phenyl]-piperidin,
4-[2-(4-Methoxy-phenoxy)-phenyl]-piperidin,
4-[2-{2-Chlor-4-methoxy-phenoxy}-phenyl]-piperidin,
4-[2-(3,4-Dimethoxy-phenoxy)-phenyl]-piperidin, und
4-[2-(4-Chlor-3-trifluormethyl-phenoxy)-phenyl]-piperidin ist,
oder ein pharmazeutisch verträgliches Salz davon.

16. Pharmazeutische Zusammensetzung, umfassend eine Verbindung nach einem der Ansprüche 1 bis 15 oder ein pharmazeutisch verträgliches Säureadditionssalz davon und zumindest einen pharmazeutisch verträglichen Träger oder Verdünner.

17. Verwendung einer Verbindung nach einem der Ansprüche 1 bis 15 oder eines pharmazeutisch verträglichen Säureadditionssalzes davon für die Herstellung eines Medikaments für die Behandlung von Affektstörungen, wie Depression, Angststörungen, einschließlich allgemeiner Angststörung, sozialer Angststörung, posttraumatischer Stressstörung, obsessiv-kompulsiver Störung, Panikstörung, Panikattacken, spezifischer Phobien, sozialer Phobien und Agoraphobie.

18. Verbindung nach einem der Ansprüche 1 bis 15 für die Verwendung als ein Medikament.

## Revendications

1. Composé représenté par la formule générale I: 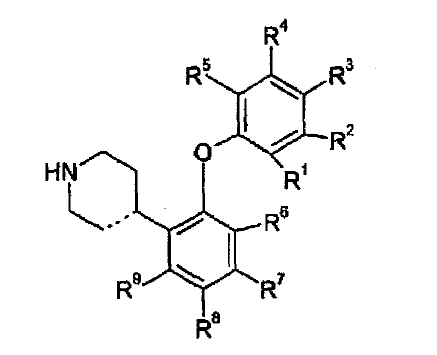 dans laquelle
la ligne pointillée ---- indique une simple liaison ou une double liaison;
R¹, R², R³, R⁴, R⁵ sont choisis indépendamment parmi: hydrogène, halogène, cyano, alk(én/yn)yle en C₁-C₆, alk(én/yn)yloxy en C₁-C₆, alk(én/yn)ylsulfanyle en C₁-C₆, hydroxy, hydroxyalk(én/yn)yle en C₁-C₆, halogénoalk(én/yn)yle en C₁-C₆, halogénoalk(én/yn)yloxy en C₁-C₆ ou -NR^{x}R^{y} où R^{x} et R^{y} sont choisis indépendamment parmi: hydrogène, alk(én/yn)yle en C₁-C₆, cyano(alk(én/yn)yle en C₁-C₆), cycloalk(én)yle en C₃-C₈, (cycloalk(én)yle en C₃-C₈)-(alk(én/yn)yle en Ci-C₆), ou NR^{z}R^{w}-(alk(én/yn)yle en C₁-C₆), où R^{z} et R^{w} sont choisis indépendamment parmi: hydrogène, alk(én/yn)yle en C₁-C₆, cycloalk(én)yle en C₃-C₈ ou (cycloalk(én)yle en C₃-C₈)-(alk(én/yn)yle en C₁-C₆); ou R^{x} et R^{y} forment ensemble, avec l'azote auquel ils sont liés, un cycle de 3 à 7 chaînons qui contient éventuellement un autre hétéroatome; ou
R² et R³ forment ensemble un hétérocycle condensé au cycle phényle choisi parmi: 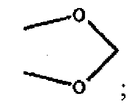 et R¹, R⁴, R⁵ sont tels que définis ci-dessus;
R⁶, R⁷, R⁸, R⁹ sont choisis indépendamment parmi: hydrogène, halogène, alk(én/yn)yle en C₁-C₆, alk(én/yn)yloxy en C₁-C₆, alk(én/yn)ylsulfanyle en C₁-C₆, hydroxy, hydroxyalk(én/yn)yle en C₁-C₆, halogénoalk(én/yn)yle en C₁-C₆, halogénoalk(én/yn)yloxy en C₁-C₆ ou -NR^{x}R^{y} où R^{x} et R^{y} sont choisis indépendamment parmi: hydrogène, alk(én/yn)yle en C₁-C₆, cyano(alk(én/yn)yle en C₁-C₆), cycloalk(én)yle en C₃-C₈, (cycloalk(én)yl en C₃-C₈)-(alk(én/yn)yle en C₁-C₆), ou NR^{z}R^{w}-(alk(én/yn)yle en C₁-C₆), où R^{z} et R^{w} sont choisis indépendamment parmi: hydrogène, alk(én/yn)yle en C₁-C₆, cycloalk(én)yle en C₃-C₈ ou (cycloalk(én)yl en C₃-C₈)-(alk(énlyn)yle en C₁-C₆); ou R^{x} et R^{y} forment ensemble, avec l'azote auquel ils sont liés, un cycle de 3 à 7 chaînons qui contient éventuellement un autre hétéroatome;
à condition qu'au moins l'un des R¹, R², R³, R⁴, R⁵, R⁶, R⁷, R⁸ et R⁹ soit différent de l'hydrogène,
ou un de ses sels.

2. Composé selon la revendication 1, dans lequel R¹ est choisi parmi: hydrogène, halogène, cyano, alk(én/yn)yle en C₁-C₆, alk(én/yn)yloxy en C₁-C₆, alk(én/yn)ylsulfanyle en C₁-C₆, halogénoalk(én/yn)yle en C₁-C₆, ou -NR^{x}R^{y} où R^{x} et R^{y} sont choisis indépendamment parmi: hydrogène, alk(én/yn)yle en C₁-C₆, cyano(alk(én/yn)yle en C₁-C₆), cycloalk(én)yle en C₃-C₈, (cycloalk(én)yl en C₃-C₈)-(alk(én/yn)yle en C₁-C₆), ou NR^{z}R^{w}-(alk(én/yn)yle en C₁-C₆), où R^{z} et R^{w} sont choisis indépendamment parmi: hydrogène, alk(én/yn)yle en C₁-C₆, cycloalk(én)yle en C₃-C₈ ou (cycloalk(én)yl en C₃-C₈)-(alk(én/yn)yle en C₁-C₆), à condition que, si l'un des R^{x} et R^{y} est NR^{z}R^{w}-(alk(én/yn)yle en C₁-C₆), l'autre soit choisi parmi: hydrogène, alk(én/yn)yle en C₁-C₆, cyano(alk(én/yn)yle en C₁-C₆), cycloalk(én)yle en C₃-C₈ ou (cycloalk(én)yl en C₃-C₈)-(alk(én/yn)yle en C₁-C₆); ou R^{x} et R^{y} forment ensemble, avec l'azote auquel ils sont liés, un cycle de 3 à 7 chaînons qui contient éventuellement un autre hétéroatome; de façon typique, R¹ est choisi parmi: hydrogène, alkyle en C₁-C₆ ou halogène.

3. Composé selon l'une quelconque des revendications 1-2, dans lequel R² est choisi parmi: hydrogène, halogène, cyano, alk(én/yn)yle en C₁-C₆, alk(én/yn)yloxy en C₁-C₆, alk(én/yn)ylsulfanyle en C₁-C₆, halogénoalk(én/yn)yle en C₁-C₆; de façon typique, R² est choisi parmi: hydrogène, alcoxy en C₁-C₆, halogénoalkyle en C₁-C₆, alkyle en C₁-C₆ ou halogène; de façon plus typique, R² est choisi parmi: hydrogène ou alcoxy en C₁-C₆.

4. Composé selon l'une quelconque des revendications 1-3, dans lequel R³ est choisi parmi: hydrogène, halogène, cyano, alk(én/yn)yle en C₁-C₆, alk(én/yn)yloxy en C₁-C₆, alk(én/yn)ylsulfanyle en C₁-C₆, halogénoalk(én/yn)yle en C₁-C₆; de façon typique, R³ est choisi parmi: hydrogène, alkyle en C₁-C₆, alcoxy en C₁-C₆, halogène ou halogénoalkyle en C₁-C₆; de façon plus typique, R³ est choisi parmi: hydrogène, alkyle en C₁-C₆, alcoxy en C₁-C₆ ou halogène.

5. Composé selon l'une quelconque des revendications 1-2, dans lequel R² et R³ forment ensemble un hétérocycle condensé au cycle phényle choisi parmi:

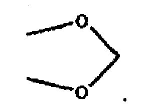

6. Composé selon l'une quelconque des revendications 1-5, dans lequel R⁴ est choisi parmi: hydrogène, halogène, cyano, alk(én/yn)yle en C₁-C₆, alk(én/yn)yloxy en C₁-C₆, alk(én/yn)ylsulfanyle en C₁-C₆, halogénoalk(én/yn)yle en C₁-C₆; de façon typique, R⁴ est choisi parmi: hydrogène, alcoxy en C₁-C₆, halogénoalkyle en C₁-C₆, alkyle en C₁-C₆ ou halogène; de façon plus typique, R⁴ est choisi parmi: hydrogène ou alcoxy en C₁-C₆.

7. Composé selon l'une quelconque des revendications 1-6, dans lequel R⁵ est choisi parmi: hydrogène, halogène, cyano, alk(én/yn)yle en C₁-C₆, alk(énlyn)yloxy en C₁-C₆, alk(én/yn)ylsulfanyle en C₁-C₆, halogénoalk(én/yn)yle en C₁-C₆, ou -NR^{x}R^{y} où R^{x} et R^{y} sont choisis indépendamment parmi: hydrogène, alk(én/yn)yle en C₁-C₆, cyano(alk(én/yn)yle en C₁-C₆), cycloalk(én)yle en C₃-C₈, (cycloalk(én)yl en C₃-C₈)-(alk(én/yn)yle en C₁-C₆), ou NR^{z}R^{w}-(alk(én/yn)yle en C₁-C₆), où R^{z} et R^{w} sont choisis indépendamment parmi: hydrogène, alk(én/yn)yle en C₁-C₆, cycloalk(én)yle en C₃-C₈ ou (cycloalk(én)yl en C₃-C₈)-(alk(én/yn)yle en C₁-C₆), à condition que, si l'un des R^{x} et R^{y} est NR^{z}R^{w}-(alk(én/yn)yle en C₁-C₆), l'autre soit choisi parmi: hydrogène, alk(én/yn)yle en C₁-C₆, cyano(alk(én/yn)yle en C₁-C₆), cycloalk(én)yle en C₃-C₈ ou (cycloalk(én)yl en C₃-C₈)-(alk(én/yn)yle en C₁-C₆); ou R^{x} et R^{y} forment ensemble, avec l'azote auquel ils sont liés, un cycle de 3 à 7 chaînons qui contient éventuellement un autre hétéroatome; de façon typique, R⁵ est choisi parmi: hydrogène, alkyle en C₁-C₆ ou halogène.

8. Composé selon l'une quelconque des revendications 1-7, dans lequel R⁶ est choisi parmi: hydrogène, halogène, alk(én/yn)yle en C₁-C₆, halogénoalk(én/yn)yle en C₁-C₆; de façon typique, R⁶ est choisi parmi: hydrogène ou halogène.

9. Composé selon l'une quelconque des revendications 1-8, dans lequel R⁷ est choisi parmi: hydrogène, halogène, alk(én/yn)yle en C₁-C₆, halogénoalk(én/yn)yle en C₁-C₆; de façon typique, R⁷ est choisi parmi: hydrogène ou halogène.

10. Composé selon l'une quelconque des revendications 1-9, dans lequel R⁸ est choisi parmi: hydrogène, halogène, alk(én/yn)yle en C₁-C₆, halogénoalk(én/yn)yle en C₁-C₆, ou -NR^{x}R^{y} où R^{x} et R^{y} sont choisis indépendamment parmi: hydrogène, alk(én/yn)yle en C₁-C₆, cyano(alk(én/yn)yle en C₁-C₆), cycloalk(én)yle en C₃-C₈, (cycloalk(én)yl en C₃-C₈)-(alk(én/yn)yle en Ci-C₆), ou NR^{z}R^{w}-(alk(én/yn)yle en C₁-C₆), où R^{z} et R^{w} sont choisis indépendamment parmi: hydrogène, alk(én/yn)yle en C₁-C₆, cycloalk(én)yle en C₃-C₈ ou (cycloalk(én)yl en C₃-C₈)-(alk(én/yn)yle en C₁-C₆), à condition que, si l'un des R^{x} et R^{y} est NR^{z}R^{w}-(alk(én/yn)yle en C₁-C₆), l'autre soit choisi parmi: hydrogène, alk(én/yn)yle en C₁-C₆, cyano(alk(én/yn)yle en C₁-C₆), cycloalk(én)yle en C₃-C₈ ou (cycloalk(én)yl en C₃-C₈)-(alk(én/yn)yle en C₁-C₆); ou R^{x} et R^{y} forment ensemble, avec l'azote auquel ils sont liés, un cycle de 3 à 7 chaînons qui contient éventuellement un autre hétéroatome; de façon typique, R⁸ est choisi parmi: hydrogène, halogénoalkyle en C₃-C₆, alkyle en C₁-C₆ ou halogène.

11. Composé selon l'une quelconque des revendications 1-10, dans lequel R⁹ est choisi parmi: hydrogène, halogène, alk(én/yn)yle en C₁-C₆, halogénoalk(én/yn)yle en C₁-C₆; de façon typique, R⁹ est choisi parmi: hydrogène.

12. Composé selon l'une quelconque des revendications 1-11, dans lequel la ligne pointillée ---- indique une simple liaison.

13. Composé selon l'une quelconque des revendications 1-11, dans lequel la ligne pointillée ---- indique une double liaison.

14. Composé selon l'une quelconque des revendications 1-13, où le composé de formule I a dans le(s) cycle(s) phényle 1-4 substituants, choisis parmi n'importe lequel des R¹-R⁹, qui sont différents de l'hydrogène, et les autres substituants sont l'hydrogène.

15. Composé selon la revendication 1, ledit composé étant
la 4-[2-(2,4-diméthylphénoxy)phényl]-1,2,3,6-tétrahydropyridine,
la 4-[2-(4-chlorophénoxy)phényl]-1,2,3,6-tétrahydropyridine,
la 4-[2-(4-fluoro-2-méthylphénoxy)phényl]-1,2,3,6-tétrahydropyridine,
la 4-[2-(4-fluorophénoxy)phényl]-1,2,3,6-tétrahydropyridine,
la 4-[2-(4-méthylphénoxy)phényl]-1,2,3,6-tétrahydropyridine,
la 4-[2-(4-méthoxyphénoxy)phényl]-1,2,3,6-tétrahydropyridine,
la 4-[2-(2,4-diméthylphénoxy)phényl]pipéridine,
la 4-[2-(4-chlorophénoxy)phényl]pipéridine,
la 4-[2-(4-fluoro-2-méthylphénoxy)phenyl]pipéridine,
la 4-[2-(4-fluorophénoxy)phényl]pipéridine,
la 4-[2-(4-méthylphénoxy)phényl]pipéridine,
la 4-[2-(4-chloro-2-méthylphénoxy)phényl]pipéridine,
la 4-[2-(3-chloro-2-méthylphénoxy)phényl]pipéridine,
la 4-[2-(2-chloro-4-méthylphénoxy)phényl]pipéridine,
la 4-[2-(2,4-dichlorophénoxy)phényl]pipéridine,
la 4-[2-(benzo[1,3]dioxol-5-yloxy)phényl]pipéridine,
la 4-[2-(4-méthoxy-2-méthylphénoxy)phényl]pipéridine,
la 4-[2-(3,4-dichlorophénoxy)phényl]pipéridine,
la 4-[2-(3,4-diméthylphénoxy)phényl]pipéridine,
la 4-[2-(2,3,4,5-tétraméthylphénoxy)phényl]pipéridine,
la 4-[2-(4-trifluorométhylphénoxy)phényl]pipéridine,
la 4-[2-(4-méthoxyphénoxy)phényl]pipéridine,
la 4-[2-(2-chloro-4-méthoxyphénoxy)phényl]pipéridine,
la 4-[2-(3,4-diméthoxyphénoxy)phényl]pipéridine, et
la 4-[2-(4-chloro-3-trifluorométhylphénoxy)phényl]pipéridine;
ou un de leurs sels pharmaceutiquement acceptables.

16. Composition pharmaceutique comprenant un composé selon l'une quelconque des revendications 1-15 ou un de ses sels d'addition d'acides pharmaceutiquement acceptables et au moins un support ou diluant pharmaceutiquement acceptable.

17. Utilisation d'un composé selon l'une quelconque des revendications 1 à 15 ou d'un de ses sels d'addition d'acides pharmaceutiquement acceptables pour la préparation d'un médicament destiné au traitement de troubles affectifs comme la dépression, des troubles d'anxiété comprenant le trouble d'anxiété généralisée, le trouble d'anxiété sociale, le trouble de stress post-traumatique, le trouble obsessionnel compulsif, le trouble de panique, les crises de panique, les phobies spécifiques, la phobie sociale et l'agoraphobie.

18. Composé selon l'une quelconque des revendications 1-15, pour son utilisation comme médicament.
